# EUROPEAN PATENT APPLICATION

(11) **EP 3 216 443 A1**
(43) Date of publication of application: **13.09.2017**
(21) Application number: 16000586.4
(22) Date of filing: 10.03.2016
(51) Int. Cl.: A61K 9/00, A47J 31/40, A47J 31/00, B65D 85/804

(54) **BEVERAGE PREPARATION CAPSULE FOR DELIVERY OF A SOLUBILISATE**

(71) Applicant: Athenion AG, 6304 Zug (CH)
(72) Inventor: Brysch, Ekkehard, 26316 Varel (DE); Brysch, Wolfgang, 13505 Berlin (DE); von Wegerer, Jörg, 13597 Berlin (DE)

(57) **Abstract**

The present invention relates to a beverage preparation capsule for the delivery of a solubilisate, a beverage dispensing system configured for the use of such a beverage preparation capsule and a method for preparing a beverage by means of such a beverage dispensing system. Poorly water-soluble dietary supplements or pharmaceutical active agents can be delivered in this new dosage form in order to increase the bioavailability of these substances.

## Description

The present invention relates to a beverage preparation capsule for the delivery of a solubilisate, a beverage dispensing system configured for the use of such a beverage preparation capsule and a method for preparing a beverage by means of such a beverage dispensing system.

A broad variety of substances are known for which potentially beneficial effects for the human health have been found in *in vitro* experiments. The use of many of them, however, has been seriously limited by the poor bioavailability that can be achieved by application forms known in the state-of-the-art. In pharmacology, bioavailability is a parameter that indicates the fraction of an administered dose of unchanged drug that finally becomes available in the systemic circulation for the desired pharmacological effects. This poor bioavailability is often due to a poor water solubility, respectively the lipophilic nature of the active agent to be administered. Hence the use of such substances as a dietary supplement or as a pharmaceutically active agent is impaired when using standard dosage forms.

There is a variety of approaches for improving the solubility of such agents and in many cases also their bioavailability by using of solubilization techniques. Herein the solubility of an agent in a medium is augmented by adding a third substance. These third substances are referred to as solubilizers (solubilizing agents), substances that may for example build a complex with the substance to be solubilized. Examples for such chelating agents are sodium benzoate and sodium salicylate. Another mechanism of action of solubilizers is the augmentation of the dissolving capacity of the solvent, for example by disturbing the cluster structure of water. Examples for such structure breakers are glycerol (glycerin) and macrogols (polyethylene glycol, PEG).

A third solubilization mechanism are micelle and liposome application technologies. They have won broad attention throughout the last decades. Herein the substance to be delivered is enclosed in a spherical aggregate of surfactant molecules. These molecules are characterized by a polar head group and a long nonpolar chain ("tail"). When given into an aqueous medium these molecules tend to associate by aggregating to spherical structures by orienting the polar head group towards the surrounding medium and the nonpolar chain towards the interior of the spheres. When these spheres consist of only one layer of such amphiphilic molecules they are referred to as micelles. Depending on the nature of the amphiphilic molecule and the reaction conditions it is also possible to form spheres with more than one layer. Herein a second layer is formed inside the outer layer of the sphere, the nonpolar groups of this second layer being oriented towards the nonpolar groups of the outer layer, and the polar head groups being oriented towards the interior of the sphere. Such aggregates are referred to as liposomes. In their structure they resemble the lipid bilayer of the cell membrane. Often the same or structurally related components are used for liposomes as those known from the cell membrane, therefore displaying similar physicochemical properties. There are also multi-layered liposomes in which at least two liposomal spheres are formed over one another, thus building a multispherical aggregate. When given in a lipophilic medium these substances tend to build inversed spherical structures where the lipophilic chain is oriented towards the solution medium and the other layers are arranged accordingly.

It is known for a long time that it is possible to enclose substances inside such spherical structures. Different uses of such loaded spheres have been described in the art, among them the usage as a dosage form for the application of lipophilic substances and/or for increasing the bioavailability of the enclosed substance. In micelles, the enclosed nonpolar substance concentrates in the interior space of the sphere toward which the nonpolar chains of the amphiphilic molecules are oriented. In liposomes, however, the interior space of the spheres is an aqueous, respectively hydrophilic medium. It can serve for packaging hydrophilic molecules. Poorly water-soluble, respectively lipophilic molecules, however, gather mostly in between the lipophilic structures of the liposomal layers.

From empiric pharmacokinetic measurements it is known that micelles as well as liposomes are absorbed from the organism in the gastrointestinal tract to a comparatively high degree, in particular from the intestinal villi. Thus a substance packed in such a spherical aggregate is absorbed to a much higher degree into the systemic bloodstream into which a certain percentage of the enclosed substance is released through different physiologic and non-physiologic mechanisms. Thus this substance becomes bioavailable and can exert its actions in the organism. If needed it can be transported via the cellular membrane to the interior of a cell. The transport, respectively the absorption rate over the cell membrane is an intrinsic characteristic for each substance, depending on a variety of factors such as molecule size, degree of lipophilicity and the presence of suitable transporter molecules inside the cell membrane. In general, lipophilic substances are transported more easily over the cell membrane because of the lipophilic nature of the lipid bilayer of the cell membrane. From liposomes it is also known that they are able to fuse with the cell membrane by invagination, thus delivering the enclosed substance to a considerable degree into the cytosol. Certain cell types, in particular phagocytes such as macrophages, monocytes and neutrophils, preferably ingest liposomes which then may undergo metabolic digestion and thus deliver the enclosed substance to these cells.

Liposomal applications have been widely discussed in medicine and pharmacology and many sophisticated technologies for their production have been developed. Their use, however, is not very common. One reason are the relatively high production costs, another reason are possible adverse side effects. In particular, when parenterally applied, liposomes carry the risk of accumulating in the liver, the spleen and/or the bone marrow. This problem occurs rather seldom in oral dosage forms.

Self-emulsifying drug delivery systems (SEDDS) are another approach to solubilize lipophilic compounds. They use to be isotropic mixtures of oils, surfactants, solvents and optionally cosolvents. Depending on the used components they may form solubilisates or stable oil-in-water (o/w) emulsions upon aqueous dilution and optionally gentle agitation.

Another solubilization technique is the formation of inclusion complexes of the substance to be solubilized with cyclodextrins such as α-, β- or γ-cyclodextrin or cyclodextrin derivatives such as 2-hydroxypropyl-β-cyclodextrin, methyl-β-cyclodextrin or trimethyl-β-cyclodextrin. Typically, cyclodextrins are composed of 6 to 8 1,4-linked α-D-glucopyranosides forming macrocycles. Thus a water-soluble toroid (respectively cone-shaped or bucket-shaped) structure is generated which is capable to host hydrophobic substances in its interior. The interior space is considerably less hydrophilic than the outside contacting the aqueous environment. Cyclodextrins are produced from starch by enzymatic treatment. They are loaded with the compound to be solubilized by dispersion. The compound to be solubilized can then be released by contacting these complexes with water, by pH or temperature changes, depending on the specific composition. Cyclodextrins are used a.o. for dietary supplements (e.g. Cavamax^{®} by Wacker Chemie, Germany) or for pharmaceutical drug delivery (e.g. for diclofenac (EP 0 658 347 A2) or clarithromycin (Allsopp, (2013), Development of a soluble macrolide formulation and identification of potential benefits in chronic rhinosinusitis MPhil Thesis, University of Queensland). If recrystallization of the compound to be solubilized occurs above a certain final concentration it may help to add an emulsifier such as a polysorbate (e.g. Tween^{®} 20; EP 1 609 481 A1).

Often it is not very comfortable to mix such solubilisates with the diluents. It is often regarded as cumbersome and weary, in particular when the mixing takes time, or stirring or agitation of the final beverage is required. A major problem is that the solubilisate often sticks to the container walls and thus is only partially released from the container into the solvent without further means. Another major problem is the appeal of such a procedure, in particular when the beverage is not prepared by medical staff but by the consumer or the patient himself. Consumers strongly prefer quick preparation methods and an easy handling. For example, opening an ampoule oneself, although relatively safe nowadays, is a strong emotional obstacle for many consumers and patients. In case of a medication this may often lead to a poor patient compliance causing a wake of consequential problems. Further, the mixing procedure is often insufficient and may result in a poorly solubilized beverage and/or an inhomogeneous beverage. Another aspect is that the solubilization process often leads to a phenomenon called zebra effect or striping caused by an incomplete dissolution of the solubilisate (or other concentrate). Such a beverage is not particularly appealing for intake.

Therefore there is a need for new, respectively alternative dosage forms of such solubilized substances for use as dietary supplement or pharmaceutically active agent that overcome the problems mentioned above.

Thus it is the task of this patent application to provide a consumer-friendly easy-to-handle and safe liquid oral dosage form for dietary supplements or pharmaceutically active agents in need to be solubilized.

### Description of the invention

Surprisingly, it was found that beverage preparation capsules containing such a solubilisate are able to solve these problems.

Thus the present invention refers to a beverage preparation capsule containing a solubilisate of at least one pharmaceutically active agent and/or a dietary supplement.

In particular, the present invention refers to a beverage preparation capsule containing a solubilisate of at least one pharmaceutically active agent and/or a dietary supplement, wherein the solubilisate is prepared from at least one poorly water-soluble substance or extract.

It is preferred that the solubilisate according to the invention is prepared by means of micelle, liposome, self-emulsification or cyclodextrin complexation technology.

According to the invention a solubilisate is the composition of the substance to be solubilized, a solvent and a solubilizing agent. It is characterized by the substantially complete solubilization of the substance, thus being a nearly perfect solution in which the molecules behave completely as independent entities in a solution and undergo the distribution and thermodynamic rules of Brownian motion. A solubilisate must be differentiated from a suspension (colloidal suspension). This term defines a heterogeneous mixture containing solid particles that sooner or later will undergo sedimentation. Therefore, micellar or liposomal solubilisates are no suspensions. It is also different from an emulsion (a mixture of two liquids which usually are immiscible). For increasing the bioavailability of a substance the complete solubilization is highly preferably. Therefore solubilisates are preferred over suspensions or emulsions.

The term solubilisate used according to the invention must be differentiated from the finished solution, respectively the prepared beverage to be imbibed. This finished solution according to the invention is generated by the preparation of the solubilisate in the beverage preparation capsule according to the invention and the concomitant, respectively subsequent release into a container such as a glass.

A solubilisate according to the invention must be also differentiated from a concentrate. A concentrate is a compound, respectively a composition of compounds without a diluent. Upon release of a concentrate into a diluent the concentrate dissolves itself either completely in the diluent or forms a suspension or emulsion with the diluent. A concentrate does not need the interaction with a solubilizing agent and/or a solvent, as it is intrinsically solvable in water or an aqueous solution. The present invention does not refer to the use of a concentrate in a beverage preparation capsule.

Some solubilisates, however, require the addition of water during the preparation process. Herein, water is a diluent and not a solvent. In some cases it may be also preferable to prepare and/or to store a solubilisate in an aqueous solution. In such an aqueous solution the concentration of the substance to be solubilized is still much higher as in the finished solution ready for drink. According to the invention the term solubilisate shall include also such aqueous solutions of solubilisates. The volume ratio of aqueous solution to finished solution is maximum 1:4, preferred 1:10 - 1:100, most preferred 1:20 - 1:50.

As used in the present patent application, the terms "aqueous solution" and "finished solution" should be clearly differentiated. "Aqueous solution" refers to the solubilisate with an amount of water added, as it is filled into the beverage preparation capsule according to the invention. "Finished solution" refers to the final beverage ready for intake by the user, i.e. the content of the beverage preparation capsule according to the invention dissolved in a diluent (in most cases water).

Finished solutions of dietary supplements or pharmaceutically active agents prepared by such a solubilization technique should display a long-term stability so that they offer a reasonable shelf-life. Otherwise they are not very attractive for producers, vendors and finally also for customers or patients. Such a long-term stability is not easy to achieve with many solubilisates, in particular in liquid dosage forms for oral administration. The alternative are ready-to-use or instant preparations in which the substance to be solubilized, the solubilizer and the solvent are freshly mixed and the resulting beverage is ingested soon after. Herein liquid concentrates or solid forms are admixed to the solvent, in general water or an aqueous solution. These liquid concentrates or solid forms can be provided in form of containers such as bottles, vials, ampoules, capsules to be opened, sachets, "tea bags", tear-open envelopes or prefilled syringes.

Beverage preparation capsules are mostly known for single-use coffee preparations. The basic form was disclosed in Nestlé's seminal patent EP 0 512 468 B1. Herein, a capsule (cartridge) is disclosed that contains a coffee, tea or chocolate powder or any other dehydrated edible substance which is going to be prepared inside the capsule and then extracted under pressure and delivered into a drinking vessel to the user. Such a capsule comprises a cup with a base and a frustoconical (truncated cone-shaped) lateral wall, a circular lip (flange) larger in diameter than the base on the side of the cup opposite to the base and a cover welded to the periphery of said lip. This cover consists of a flexible material, for example aluminium, other thin metal foils, single- or multi-layered plastics, cardboard, paper and composite materials thereof. The same materials can be used for said cup. Preferably, the cup and cover materials are impermeable for oxygen and moisture. This cover is not intended to be torn open by the user but to yield only under the pressure of the extraction fluid applied on said cover when starting the extraction of the beverage. Preferably, the cup has a thickness of 20 to 100 µm. The cover has preferably a thickness of 15 to 60 µm. When using a composite or multi-layer material the thickness of the respective layers sums up correspondingly to stay inside these boundaries. Preferably, the capsule itself has a diameter of 25 to 60 mm and preferably a height (distance between base and cover) of 10 to 25 mm. Preferred plastic materials are EVOH (ethylene vinyl alcohol copolymer), PVDC (polyvinylidene chloride), PP (polypropylene), PE (polyethylene), PA (polyamide) or PET (polyethylene terephthalate), or functionalized plastic materials such as metallized PET or PET with a high-performance barrier layer from for example SiO₂.

Since then beverage preparation capsules have been developed further. There exist single-serve hot beverage systems using preferentially, but not exclusively coffee capsules, for example from Nestlé (Dolce Gusto, Nespresso, Nespresso VertuoLine, Special T), Lavazza (Espresso Point Espresso Point MAXI, Blue, A Modio Mio, Brita Yource), Bialetti (Bialetti Diva), Tchibo, Kenco Singles, Krafft (Tassimo, T-Discs), Coffea, Bodecker Brewed, Caffita (Caffitaly), Delta Cafés (Delta Q), Italian ESE (Easy Serving Espresso Pod), Mars (Flavia Beverage Systems), Tuttoespresso, Hausbrandt Trieste, Folgers, Illy (iperEspresso), Keurig (Green Mountain Coffee Roasters), Aldo Espressi (K-Fee), Sara Lee (Senseo), Starbucks (Verismo); capsule-based systems particularly designed for commercial purposes such as Esio, PHSI (Interpure), Waterlogic (Innowave), Vertex (Charm), Mars (Flavia); capsule-based systems for cold beverages have been introduced to the market for example from Omnifrio, Promo Water, Bevyz, Esio, Brita (Yource), Keurig.

The basic structure and *modus operandi* of all these capsules (cartridges, pods) is similar, although there exist differences in details. For example, also the base of the cup may consist of a flexible foil. In the art, the cover and/or base foil is also named a membrane, diaphragm, film or seal foil.

The cover and /or the base of the cup may be perforated not only by pressure but also by a cutting tool, respectively a puncher linked to the beverage dispensing machine. This way an opening is generated through the membrane, diaphragm, film or seal foil that allows either a temporally and/or spatially controlled release of the at least one supplement, or a complete release at once. Such a cutting tool (perforator) is disclosed in WO 92/07775 A1, wherein this perforator is a hollow cylinder with a sharp tip through the lumen of which the brewing water is applied into the capsule.

From the art capsules with an inset are known wherein this inset is arranged at the lower bottom (the base of the cup), for example as an extra piece, mostly from plastic or as a unit with the cup, wherein the spikes of this inset are pushed against the lower foil, respectively the base of the cup in order to perforate it upon application of pressure inside the capsule and thus allow the coffee to be released through these perforation holes into a cup, respectively container, preferably through an outlet tube (cf. WO 92/07775 A1). In some capsule embodiments the lower foil, respectively the base of the cup or sealing member is pressed against a matching pressing surface of the beverage dispensing machine, preferably also provided with spikes or the like. For this operational mode said sealing member should be resilient, respectively elastic. Herein the perforation occurs from the outside (cf. WO 2006/045536).

A variation of this capsule form is disclosed in WO 2004/087529 A1. Herein an inset is realized as an end wall with orifices, this inset being a part of the plastic casing and separating the capsule interior with the coffee powder from an underlying container. The prepared beverage is pressed through these orifices like through a percolator.

In WO 2005/018395 A1 a capsule with an inset is described that likewise separates the chamber with the coffee powder from an underlying dispensing chamber. Through an interaction of the variably applied pressure (in total between 1.2 and 8 bars), the thickness of the upper foil (cover) and the specific dentate structure, respectively protrusions of the percolator inset the amount of produced foam can be regulated, depending on the chosen capsule. For example, different operational parameters and/or insets are chosen for coffee-containing capsules and milk powder-containing capsules for cappuccino. The outlet from the lower container can be controlled via a baffle provided with openings and positioned on the base of the cup, thus slowing the outflow of the prepared beverage from the lower container.

In another capsule from the art there is a distributing device tethered to the lower surface of the cover (foil) which shall effect a better mixing of the incoming pressurized water with the coffee powder inside the capsule. This device can be combined with a screening device tethered at the bottom (the base) of the capsule. The inlet of the pressurized water into the capsule and/or the outlet of the thus prepared beverage is effected through these distributing and/or screening devices. They are provided with some openings (holes) through which the liquid is going to flow. These openings may be provided with a textile that acts as a filter (cf. US 2006/0236871).

In WO 2005/080223 A1 a capsule is disclosed which comprises means for varying the area for the outlet of the prepared beverage from the capsule. Preferred means are hinges. They serve to impart an oscillating movement to this lower wall during the dispensing step.

WO 2005/092160 A1 and EP 2343247 A1 disclose mold-cast capsules in which a dispenser means in form of a hollow cylinder perforates the cover of the capsule, allowing the pressurized water to enter the coffee powder-filled cup interior. The prepared beverage, however, has to dam up inside the cup until it can evade via a hollow raised portion molded to the base of the cup. After flowing through the lumen of said raised portion it perforates the lower foil at the base of the cup via fluid pressure. Thus an improved mixing of the pressurized water and the beverage powder, respectively an increased homogeneity of the prepared beverage shall be effected.

The cutting tool can have the shape of a puncher, a stamp, a blade, a knife, a cutting disc, a scissor, a milling cutter, a drill, a chisel. It can be driven vertically or in a beveled angle through the material to be cut. It can be made of metals, medical stainless steel, alloys, rigid plastics, glass, ceramics, diamond, boron nitride. The at least one membrane, diaphragm, film or seal foil can be punched, pierced or cut as a whole or compartment-, respectively sector-wise. Alternatively, said cutting tool can cut or punch through a predetermined weak spot in the capsule. Such a weak spot can be generated by default during mold-cast.

In another preferred embodiment the at least one membrane, diaphragm, film or seal foil is pre-punctured, thus enabling, respectively facilitating a controlled cutting line on operating said cutting tool.

This at least one membrane, diaphragm, film or seal foil can be made of metal (such as aluminium foil, tin foil, silver foil, gold foil, copper foil), a polymer (such as polyolefins, polyethylene, polypropylene, polyvinylchloride, polystyrene, polycarbonate, cellophane, cellulose esters such as cellulose acetate and nitrocellulose, polylactic acid, polyester, in particular polyhydroxyalkanoates such as poly-3-hydroxybutyrate, polyhydroxyvalerate and polyhydroxyhexanoate, polyamide 11, polyethylene terephthalate, starch blends, parafilm), paper (such as writing paper, rolling paper, banana paper, waterproof paper, parchment paper, greaseproof paper, wax paper, cardboard, wrapping tissue), a rubber stopper material (such as canonized in current pharmacopoeias) or a composite material produced from the aforementioned materials. The advantage of these improved foils is a better isolation of the content of the beverage preparation capsule from oxygen, UV irradiation and/or heat.

In preferred embodiments the thickness of a metal foil is less than 20 µm, more preferred less than 18 µm, most preferred less than 15 µm.

In preferred embodiments the ultimate tensile strength of a metal foil is in the range of 20 - 220 N/mm², more preferred between 30 - 160 N/mm², even more preferred between 40 - 125 N/mm², most preferred between 45 - 95 N/mm².

In preferred embodiments the thickness of a polymer-based foil is less than 100 µm, more preferred less than 80 µm, most preferred less than 50 µm.

In preferred embodiments the ultimate tensile strength of a polymer-based foil is in the range of 20 - 300 N/mm², more preferred between 40 - 250 N/mm², even more preferred between 60 - 200 N/mm², most preferred between 80 - 180 N/mm².

In preferred embodiments the thickness of said paper is less than 200 µm, more preferred less than 160 µm, most preferred less than 120 µm.

In preferred embodiments the bursting strength of said paper is in the range of 100 - 700 KPa, more preferred between 150 - 500 KPa, even more preferred between 200 - 400 Kpa, most preferred between 250 - 300 KPa.

For embodiments in need of a particularly tight-sealing foil (for example for excluding oxygen entry to oxidation-sensitive supplements such as vitamin C) metal foils are preferred.

One problem of the use of the aforementioned materials is the degradation after use, particularly in the environment. Many of these materials are very poorly degradable under natural conditions and may contribute over years to the environmental load. Moreover, many of the plastic-based materials are derived from petroleum. In the light of resource scarcity and the power consumption needed for their production this is not always desirable. Therefore in preferred embodiments non-toxic biodegradable materials are used. Moreover, their energy balance is more favorable. Such preferred foil materials are cellulose acetate, nitrocellulose, polylactic acid, poly-3-hydroxybutyrate, polyhydroxyvalerate, polyhydroxyhexanoate, polyamide 11, starch blends.

Another problem is that on cutting or punching the foil often it can't be completely avoided that tiny debris of the foil gets into the liquid in the container and thus is likely to be imbibed by the consumer. Though there are no long-term studies about health hazards of such foil (cover) debris it is certainly preferable to avoid or minimize such risks by a) using pressure mechanisms or cutting tools that generate only minimal amounts of debris on being used, b) the use of biodegradable materials that can be relatively quickly degraded by the organism, either by gastric acid or by aerobic or anaerobic bacteria in the intestinal tract (see above), or c) by biocompatible materials that will pass unchanged through the intestinal tract and don't accumulate in the organism.

A preferred example for such a biocompatible foil material is polypropylene.

In preferred embodiments a combination of the at least one membrane, diaphragm, film or seal foil and pressure application mode, respectively cutting tool is chosen that ensures that virtually no debris gets into the beverage preparation capsule, respectively the prepared beverage.

All these beverage preparation capsules are configured as to fit into a beverage dispensing system. In many cases, producers have aligned the capsules and the dispensing machine in such a way that only this particular combination works together. This exclusive compatibility is achieved by a specific size and shape of the beverage preparation capsules, and on the other hand by corresponding specific configurations of the beverage dispensing machine in respect of the receiving parts into which the beverage preparation capsule must fit and/or the insertion mechanism by which the beverage preparation capsule is inserted and/or brought into the right position to be ready for operating the dispensing or preparing mechanism. Because of ending patent protection and corresponding anti-trust court decisions in several key countries, however, there is an increasing number of competitors on the market that produce either beverage preparation capsules or beverage dispensing machines alone that are compatible with brand machines or capsules.

There is also a broad variety of beverage dispensing machines apt for receiving beverage preparation capsules, often with sophisticated extra features. Common features of such beverage dispensing machines are:
a container for providing the liquid needed for preparing a beverage with the ingredients from said capsules. In most cases this liquid is selected from tap water, carbonated tap water, mineral water, carbonated mineral water and deionized water. An alternative to such a container is a permanent liquid supply on demand, for example a standard water pipe connected to the beverage dispensing machine via a hose or a lock-in joint;
optionally, means for carbonating the liquid for preparing the beverage in the liquid storage container, or a special compartment for this purpose (a container or a tube). Such means can be for example a pressurized CO₂ cartridge, connected to said container or compartment via a hose or tube and operated by default or optionally when using the beverage dispensing system;
optionally, means for heating the liquid inside this container, alternatively in an extra compartment. This can be a boiler, a heating coil, a heating pipe, a calorifier, an immersion heater, a heat exchanger, a thermoblock, a cartridge heater, a microwave device or any other suitable heating device. Optionally, the means for heating can be switched off by the operator or via an interactive control program;
means for injection of the pressurized liquid into the beverage preparation capsule. This is often realized as an injection head, in general provided with an injection intruding part for focusing the liquid onto the beverage preparation capsule. In some embodiments this can be an injection needle;
means for pressurizing the liquid from said container or permanent liquid supply. This includes a water circuit for circulating the water from the container to the means for injection. In the water circuit the water is transported under pressure by means of a water pump. This pump can be a peristaltic pump, a piston pump, a diaphragm pump or any other suitable pumping system known from the art;
optionally, a steam supply circuit, if means for heating are provided. It comprises a second water pump for transporting the water from the container to a steam generator. The water circuit and the steam supply circuit usually converge at an intersection point located upstream to the means for injection. There a directing valve is provided. It serves for selectively directing water or steam into the connecting part with the means for injection. In this connecting part downstream of the valve a single fluid is generated from the water and the steam. This valve (e.g. a three-way valve) can be a solenoid valve, a ceramic valve, an insulation or membrane valve or any other suitable valve known in the art;
a cavity, respectively a three-dimensional recess for receiving a beverage preparation capsule;
a holder into which the beverage preparation capsule is inserted and positioned under the injection head inside the cavity. This holder can be tightly fixed to the back wall of said cavity or it can be removed, either for cleaning or for receiving a beverage preparation capsule before being reinserted again. This holder can be a part with a simple recess dimensioned for receiving a beverage preparation capsule, or it can be a revolver disk-shaped part with a plurality of recesses for receiving up to a corresponding number of beverage preparation capsules of the same or of a different content, each capsule thereof able to be positioned under the injection head either by the operator or mechanically by a control mechanism;
optionally, outlet means for letting the prepared beverage flow from the beverage preparation capsule into a drinking or storage vessel such as a glass, cup etc. This can be a collector means such as a funnel-shaped part below the inferior side of the beverage preparation capsule, combined with an outlet such as a tube or a hose through which the prepared beverage flows by means of gravity;
a second cavity, respectively a second three-dimensional recess below the outlet means into which the drinking or storage vessel can be placed in such a way that the prepared beverage can flow via the outlet means into the drinking or storage vessel. This second cavity is often an extension of the first cavity.

It is understood that suitable tubes and/or hoses between these parts as well as the necessary motors and control devices are included too in beverage dispensing machines apt for an inventive use.

According to the invention all listed embodiments of beverage preparation capsules and/or beverage dispensing machines can be combined without limitation among each other, in as far as the parts functionally match.

It should be made clear that the present invention does not refer or is limited to a specific beverage preparation capsule system and/or a specific beverage dispensing machine but should be understood as being compatible with all these systems known in the art.

The internationally accepted BCS (Biopharmaceutical Classification System) classifies drug substances into four classes: Class 1 (high solubility - high permeability), Class 2 (low solubility - high permeability), Class 3 (high solubility - low permeability and Class 4 (low solubility - low permeability).

Herein the term solubility refers to the highest dose strength that is subject to an FDA biowaiver request. A drug is classified as highly soluble when the highest dose strength is soluble in 250 ml or less of aqueous media over the pH range of 1 - 7.5. Correspondingly, drug substances that can't be solubilized that way are classified as poorly soluble.

Herein the term permeability refers to the extent of absorption of a drug in humans across the intestinal membrane (mucosa). According to the established definition a drug is classified as highly permeable if 90% or more of the orally administered dose are resorbed in the gastrointestinal tract. Correspondingly, a drug having an absorption rate of less than 90% is classified as low permeable.

Thus solubility and permeability are intrinsic substance properties. Resorption and bioavailability, however, describe pharmaceutic parameters that may be improved by suitable measures. While resorption refers to the fraction from the orally applied substance amount that is absorbed from the gastrointestinal tract the bioavailability of a substance depends not only from resorption but also from protein binding in blood and from pharmacokinetic parameters such as first-pass metabolism.

According to the invention in a preferred embodiment pharmaceutical drugs having a poor solubility as defined above are used for the production of a solubilisate.

According to the invention it is preferred that pharmaceutical drugs having a poor permeability as defined above are used for the production of a solubilisate.

According to the invention it is particularly preferred that pharmaceutical drugs having a poor solubility as well as a poor permeability as defined above are used for the production of a solubilisate (Class 4 compounds).

Examples for Class 4 pharmaceutical drugs, without being limiting, are: acetaminophen (paracetamol), acyclovir, azathioprine, azithromycin, calcitriol, carisoprodol, cefdinir, cefixime, cefuroxime axetil, cephalexin, chlorothiazide, chlorthalidone, clarithromycin, cyclosporine, dapsone, dexamethasone, dronabinol, dutasteride, furosemide, glipizide, griseofulvin, hydrochlorothiazide, indinavir sulfate, isradipine, linezolid, loperamide, mebendazole, mercaptopurine, mesalamine, methylprednisolone, modafinil, nabumetone, nelfinavir mesylate, norelgestromin, nystatin, oxcarbazepine, oxycodone HCl, progesterone, pyrimethamine, ritonavir, spironolactone, sulfamethoxazole, trimethoprim, taladafil.

For dietary supplements, the term bioavailability is used slightly differently. In most cases they are consumed orally. Thus this term defines the quantity or fraction of the ingested dose that is absorbed.

According to the invention it is preferred that dietary supplements having a poor bioavailability are used for the production of a solubilisate. It is preferred that their bioavailability is less than 50%, more preferred less than 40%, more preferred less than 30%, even more preferred less than 20%, particularly preferred less than 15% and most preferred less than 10%.

Examples for compounds or plant extracts used as dietary supplements known to have a poor bioavailability are, without being limiting: flavones, flavonols, flavon-3-ols, flavonones, flavonoids, resveratrol, turmeric, curcumin, curcuminoids, demethoxycurcumin, bisdemethoxycurcumin, bis-o-demethyl curcumin, quercetin, ellagic acid, naringenin, betulin, betulinic acid, folic acid (folate), ubiquinone (Q10, coenzyme Q), glutathione, eicosapentaenoic acid (EPA), docosahexaenoic acid (DHA), uridine, chromium dichloride, L-carnitine, ursolic acid, catechin, epicatechin, epigallocatechin (EGC), epigallocatechin gallate (EGCG), epicatechin gallate (ECG), polyphenols, berberin, melatonin, polydatin, isoflavones, liposoluble vitamins A (retinol, retinal), D, E (tocopherols), F, K, α- and β-keto-boswellic acid, L-tryptophan, 5-hydroxytryptophan, L-glycine, inositol, β-carotene, tocotrienols, ascorbyl palmitate, lecithin, lutein, luteolin, lycopene, zeaxanthin, β-cryptoxanthin, red clover, saw palmetto lipid extract, ω-3 fatty acids, steroidal terpenes, non-steroidal terpenes, terpenoids; saponins, sapogenins, diosgenin, *Dioscorea spec.* extract, *Dioscorea villosa* extract, protodioscin, *Tribulus terrestris* extract, essential oils, hypericin, xanthorhizol, pyrogallol, genistein, wogonin, morin, kaempferol, *Bacopa monneri* extract, bacopin, bacoside A, bacoside A3, bacoside B, xanthorhizol, ginseng extract, *Gingko biloba* extract, pycnogenol, capsaicin, *Rubia cordifolia* extract, *Lawsennia iermis,* extract, *Aloe vera* extract, piperin, α-lipoic acid, bromelain, phlorizin, crocin, crocetin, bioperine, acerola, proanthocyanidins, anthocyanidins, aglycones of anthocyanins silibinin, silymarin, gingerols, ceramides, isoprene, prenol, isovaleric acid, geranyl pyrophosphate, eucalyptol, limonene, pinene, farnesyl pyrophosphate, artemisinin, bisabolol, geranylgeranyl pyrophosphate, phytol, taxol, forskolin, aphidicolin, squalene, lanosterol, oils, such as shark or other cartilaginous fish oils, vegetable oils, or oils from amaranth seed, rice, wheat germ or olives; squalenes, retinoids, tannins, cinnamic acid, lignins, as well as phytosterols such as β-sitosterot laurate ester, α-sitosterol laurate ester, γ-sitosterol laurate ester, campesterol myristearate ester, stigmasterol oleate ester, campesterol stearate ester, β-sitosterol oleate ester, β-sitosterol palmitate ester, β-sitosterol linoleate ester, α-sitosterol oleate ester, γ-sitosterol oleate ester, β-sitosterol myristearate ester, β-sitosterol ricinoleate ester, campesterol laurate ester, campesterol ricinoleate ester, campesterol oleate ester, campesterol linoleate ester, stigmasterol linoleate ester, stigmasterol laurate ester, stigmasterol caprate ester, α-sitosterol stearate ester, γ-sitosterol stearate ester, α-sitosterol myristearate ester, γ-sitosterol palmitate ester, campesterol ricinoleate ester, stigmasterol ricinoleate ester, campesterol ricinoleate ester, β-sitosterol, α-sitosterol, γ-sitosterol, campesterol, stigmasterol, and stigmasterol stearate ester; extracts from adaptogenic plants such as *Eleutherococcus senticosus* (Siberian ginseng, eleuthero, ciwujia), *Rhodiola rosea* (rose root), *Schisandra chinensis* (five flavor berry), *Panax ginseng* (ginseng), *Gynostemma pentaphyllum* (Jiao Gu Lan), *Morinda citrifolia* (noni, Indian mulberry), *Lentinula edodes* (shiitake), *Ganoderma spec.* (reishi, lingzhi mushroom) such as *Ganoderma lucidum, Ganoderma tsugae* and *Ganoderma sichuanense, Grifola frondosa* (maitake mushroom, hen-of-the-woods), *Agaricus spec.* (almond mushroom) such as *Agaricus subrufescens* and *Agaricus blazei Murill, Withania somnifera* (ashwagandha, winter cherry), *Ocimum tenuiflorum* (tulsi, holy basil), *Lepidum meyenii* (maca), *Andrographis paniculata* (kalmegh), *Cannabis sativa* (marihuana), *Tabebuia impetiginosa* (lapacho), *Astragalus membranaceus* (astragalus, tragacanth).

It is empirically known that poorly water-soluble pharmaceutically active agents or dietary supplements achieve an improved resorption and/or bioavailability upon being solubilized by means of a suitable method. Therefore the present application refers also to a beverage preparation capsule, in which the solubilisate of the at least one dietary supplement and/or pharmaceutically active agent enhances the resorption and/or bioavailability of at least one of said dietary supplements or pharmaceutically active agents.

Selected dietary supplement and/or pharmaceutically active agents have been solubilized by means of the aforementioned solubilizing techniques, rendering solubilisates of these substances (see Examples 1 to 10). Thus the present application refers also to a beverage preparation capsule, in which the solubilisate was prepared from a substance selected from a group comprising ß-carotene, melatonin, folic acid and quercetin, if the substance is a dietary supplement, or selected from a group comprising furosemide, acetaminophen, glipizide and clarithromycin, if the substance is a pharmaceutically active agent. A further aspect of the invention is that some pharmaceutical drugs or dietary supplements intrinsically have a bitter or unpleasant taste. In case of pharmaceutical drugs this may seriously impair patient compliance, in case of dietary supplements such a taste may be a serious commercialization obstacle. A solubilisate according to the invention can significantly help to mask this bitter or unpleasant taste by caging the substance. Micelle, liposome or self-emulsifying solubilisates use to have a neutral taste, cyclodextrin-based solubilisates a rather sweetish taste.

Thus the present invention relates also to a beverage preparation capsule containing a solubilisate of at least one pharmaceutical drug or dietary supplement in which a bitter or unpleasant taste of the at least one pharmaceutical drug or dietary supplement is masked by the solubilisate prepared by micelle, liposome, self-emulsification or cyclodextrin complexation technology.

Examples of pharmaceutical drugs with a bitter or unpleasant taste comprise, without being limiting, acetaminophen, albuterol, aminoguanidine hydrochloride, aminophylline, amitriptyline, amoxicillin trihydrate, ampicillin, amlodipine besylate, aspirin, azithromycin, barbiturates, berberin chloride, caffeine, calcium carbonate, calcium pantothenate, cephalosporins, cetirizine, chloramphenicol, chlordiazepoxide, chloroquine, chlorpheniramine, chlorpromazine, cimetidine, ciprofloxacin, clarithromycin, codeine, demerol, dextromethorphan, digitoxin, digoxin, diltiazem hydrochloride, diphenhydramine, diphenylhydantoin, doxazosin mesylate, doxylamine succinate, eletriptan, enoxacin, epinephrine, erythromycin, ethylefrine hydrochloride, etinidine, famotidine, fluconazole, glipizide, guaifenesin, ibuprofen, indeloxazine hydrochloride, lidocaine, lomotil, loratadine, lupitidine, magnesium oxide, meclizine, methacholine, morphine, neostigmine, nifentidine, niperotidine, nizatidine, ofloxacin, paracetamol, pefloxacin, penicillin, phenobarbital, phenothiazine, phenylbutazone, phenylpropanolamine, pipemidic acid, pirbuterol hydrochloride, piroxicam, prednisolone, propranolol hydrochloride, pseudoephedrine, pyridonecarboxylic acid antibacterials, ranitidine, roxatidine, salicylic acid, sertraline hydrochloride, sildenafil, spironolactone, sulbactam sodium, sulfonamides, sulfotidine, sulpyrine, sultamicillin tosylate, tenidap, terfenadine, theophylline, trimethoprim, tuvatidine, valdecoxib, zaltidine, and zonisamide.

In a preferred embodiment the solubilisate in the beverage preparation capsule contains a BCS Class 4 pharmaceutical drug with a bitter or unpleasant taste. Suitable examples comprise acetaminophen (paracetamol), azithromycin, clarithromycin, glipizide and trimethoprim.

Many dietary supplements have also a bitter or unpleasant taste, in particular many phytochemicals such as alkaloids, tannins, phenolic or polyphenolic compounds, flavonoids, isoflavones, isoflavone glucosides, glucosinolates, isothiocyanates, cucurbitacins, oxygenated tetracyclic triterpenes.

A further aspect is that some solubilisates may stick to the inner walls of the beverage preparation capsule to a certain degree and are not a 100% released upon preparing a beverage from one of the preparation capsules according to the invention. This depends mainly on the combination of drug to be solubilized and the used solubilization technique as well as of the material of the beverage preparation capsule and its specific shape. This problem can be overcome or at least widely mitigated by applying a non-stick coating film onto the inner walls of the beverage preparation capsules. This non-stick coating film should be inert, biocompatible and should not disintegrate or detach at common preparation temperatures for hot beverages (heat-resistant). Useful examples for such a non-stick coating film are teflons such as PTFE (polytetrafluoroethylene), FEP (fluorinated ethylene propylene copolymer), PFA (perfluoroalkoxy) and ETFE (ethylene tetrafluoroethylene copolymer); anodized aluminium and silicones.

Suitable techniques for applying such a non-stick coating film onto the inner walls of beverage preparation capsules include anodizing, dip spinning, cathodic dip painting, nanocoating, wet painting, powder coating, zinc thermal diffusion, polymer coating, thermal spraying, drum spraying, vacuum coating, chrome substitute and plasma deposition. It is among the knowledge of a person skilled in the art to optimize the precise process parameters.

Thus the present application refers also to a beverage preparation capsule, in which the internal surface of said capsule is partially or completely covered with a non-stick coating film.

The aforementioned solubilisates of pharmaceutical drugs or dietary supplements alone or in combination can be combined with a variety of excipients and/or additives in the beverage preparation capsules according to the invention, as laid out in the following:
Suitable vitamins are for example vitamin C (L-ascorbic acid, sodium L-ascorbate, calcium L-ascorbate, potassium L-ascorbate, L-ascorbyl 6-palmitate), vitamin A (retinol, retinyl acetate, retinyl palmitate, beta-carotene), vitamin D (cholealciferol, ergoalciferol), vitamin E (D-alpha-tocopherol, DL-alpha-tocopherol, D-alpha-tocopheryl acetate, DL-alpha-tocopheryl acetate, D-alpha-tocopheryl succinate), vitamin K (phylloquinone), vitamin B1 (thiamin hydrochloride, thiamin mononitrate), vitamin B2 (riboflavin, sodium riboflavin 5'-phosphate), niacin (nicotinic acid, nicotinamide), pantothenic acid (calcium D-pantothenate, sodium D-pantothenate, D-panthenol), vitamin B6 (pyridoxine hydrochloride, pyridoxine 5'-phosphate), folic acid (pteroyl monoglutaminic acid), vitamin B12 (cyanocobalamine, hydroxocobalamine), biotin (D-biotin).

Suitable minerals to be included are for example calcium (calcium carbonate, calcium chloride, citric acid calcium salt, calcium gluconate, calcium glycerophosphate, calcium lactate, ortho-phosphoric acid calcium salt, calcium hydroxide, calcium oxide), magnesium (magnesium acetate, magnesium carbonate, magnesium chloride, citric acid magnesium salt, magnesium gluconate, magnesium glycerophosphate, ortho-phosphoric acid magnesium salt, magnesium lactate, magnesium hydroxide, magnesium oxide, magnesium sulfate), iron (iron carbonate, iron citrate, iron ammonium citrate, iron gluconate, iron fumarate, iron sodium diphosphate, iron lactate, iron sulfate, iron diphosphate, ferric saccharate, elemental iron), copper (copper carbonate, copper citrate, copper gluconate, copper sulfate, copper lysine complex), iodine (sodium iodide, sodium iodate, potassium iodide, potassium iodate), zinc (zinc acetate, zinc chloride, zinc citrate, zinc gluconate, zinc lactate, zinc oxide, zinc carbonate, zinc sulfate), manganese (manganese carbonate, manganese chloride, manganese citrate, manganese gluconate, manganese glycerophosphate, manganese sulfate), sodium (sodium bicarbonate, sodium carbonate, sodium chloride, sodium citrate, sodium gluconate, sodium lactate, sodium hydroxide, ortho-phosphoric acid sodium salt), potassium (potassium bicarbonate, potassium carbonate, potassium chloride, potassium citrate, potassium gluconate, potassium glycerophosphate, potassium lactate, potassium hydroxide, ortho-phosphoric acid potassium salt), selenium (sodium selenite, sodium hydrogen selenite, sodium selenite), chrome (chrome-(III)-chloride, chrome-(III)-sulfate), molybdenum (ammonium molybdate (molybdenum (VI), sodium molybdate (molybdenum (VI)), fluorine (sodium fluoride, potassium fluoride), chlorine, phosphor.

Trace elements are dietary minerals that are needed by the organism in very small amounts for growth, development and physiology, for example as co-enzymes. Some of them are virtually always present in the organism in sufficient quantities, others have to be substituted in persons in need thereof. They can be selected from the group comprising chrome, cobalt, iron, iodine, copper, manganese, molybdenum, selenium, zinc, fluoride, silicon, arsenic, nickel, rubidium, tin, vanadium. They can be substituted either as a pure element or in any of the mineral forms mentioned above.

Stimulants are often and worldwide used in drinks. According to the World Health Organization (WHO) this term refers to any kind of substances increasing, accelerating or improving neuronal activity. These substances have often a psychomimetic effect. Most popular stimulants include xanthines such as caffeine, theophylline and theobromine. Guaraná contains the aforementioned xanthines. A further popular stimulant is nicotine, respectively nicotinic acid. However, there is a broad group of stimulants that in many countries are banned by law, expected to be banned in the near future, or underlie a strict regulation of health authorities, needing the prescription of a physician. This is due to their dependence potential and other hazards to consumers' health, attention deficits in traffic etc., or negative effects on social life. Thus group includes a.o. amphetamine and its derivatives, a group of piperazine derivatives, cocaine and drugs for the treatment of narcolepsy and attention deficit hyperactivity disorder (ADHD). Hence the use of this group of substances according to the invention may be possible too. Preferred is the use of caffeine.

Suitable antioxidants can be selected from the group comprising lactic acid, ascorbic acid, sodium ascorbate, calcium ascorbate, potassium ascorbate, fatty acid esters of ascorbic acid, ascorbyl palmitate, ascorbyl stearate, tocopherols, alpha-tocopherol, gamma-tocopherol, delta-tocopherol, propyl gallate, octyl gallate, dodecyl gallate, ethyl gallate, guaiac resin, erythorbic acid, sodium erythorbate, erythorbin acid, sodium erythorbin, tert-butylhydroquinone, butylated hydroxyanisole, butylated hydroxytoluene, mono-, di-, trisodium phosphate, mono-, di-, tripotassium phosphate, anoxomer, ethoxyquin, potassium lactate, stannous chloride, sodium thiosulfate, 4-hexylresorcinol, glucose oxidase.

Suitable acidity regulators can be selected from the group comprising acetic acid, potassium acetate, sodium acetate, sodium diacetate, calcium acetate, carbon dioxide, malic acid, fumaric acid, sodium lactate, potassium lactate, calcium lactate, ammonium lactate, magnesium lactate, citric acid, mono-, di-, trisodium citrate, mono-, di-, tripotassium citrate, mono-, di-, tricalcium citrate, tartaric acid, mono-, disodium tartrate, mono-, dipotassium tartrate, sodium potassium tartrate, ortho-phosphoric acid, lecithin citrate, magnesium citrate, ammonium malate, sodium malate, sodium hydrogen malate, calcium malate, calcium hydrogen malate, adipic acid, sodium adipate, potassium adipate, ammonium adipate, succinic acid, sodium fumarate, potassium fumarate, calcium fumarate, ammonium fumarate, 1,4-heptonolactone, triammonium citrate, ammonium ferric citrate, calcium glycerophosphate, isopropyl citrate, potassium carbonate, potassium bicarbonate, ammonium carbonate, ammonium bicarbonate, magnesium carbonate, magnesium bicarbonate, ferrous carbonate, ammonium sulfate, aluminium potassium sulfate, aluminium ammonium sulfate, sodium hydroxide, potassium hydroxide, ammonium hydroxide, magnesium hydroxide, gluconic acid.

Acidifiers use to be inorganic chemicals that either produce or become acid. Suitable examples are: Ammonium chloride, calcium chloride.

Often pharmaceutically active substances or dietary supplements are provided as a salt. For pharmaceutically active substances the pharmaceutically acceptable salts are listed in the respective pharmacopoeias. Thus they can be selected from the group comprising as cationic salts the respective sodium, potassium, calcium, lithium, magnesium salts, as anionic salts the respective chloride, bromide, sulfate, phosphate, acetate, citrate, oxalate, malonate, salicylate, p-aminosalicylate, malate, fumarate, succinate, ascorbate, maleate, sulfonate, phosphonate, perchlorate, nitrate, formate, propionate, gluconate, digluconate, lactate, tartrate, hydroxy maleate, pyruvate, phenyl acetate, benzoate, p-aminobenzoate, p-hydroxybenzoate, dinitrobenzoate, chlorbenzoate, mesylate, ethanesulfonate, nitrite, isethionate, ethylene sulfonate, tosylate, naphthyl sulfonate, 4-amino-besylate, camphorsulfonate, alginate, caprate, hippurate, pectinate, phthalate, quinate, mandelate, o-methyl mandelate, hydrogen besylate, picrate, cyclopentanepropionate, D-o-toluyl tartrate, tartronate, besylate, alpha-methyl benzoate, (o, m, p-)methyl benzoate, naphthylamine sulfonate, cinnamate, acrylate, trifluoroacetate, isobutyrate, phenyl butyrate, heptanoate, xylyl sulfonate, adipate, aspartate, bisulfate, borate, butyrate, camphorate, dodecylsulfate, laurate, glucoheptonate, glycerylphosphate, hemisulfate, hexanoate, hydroiodide, 2-hydroxy-ethanesulfonate, lactobionate, lauryl sulfate, nicotinate, oleate, palmitate, pamoate, persulfate, 3-phenyl propionate, pivalate, stearate, thiocyanate, undecanoate, valerate. It is understood that these salts can also be used in preparations of dietary supplements used in the present invention.

The term "pharmaceutical excipients" refers to natural or synthetic compounds that are added to a pharmaceutical formulation alongside the pharmaceutical active agent. They may help to bulk up the formulation, to enhance the desired pharmacokinetic properties or the stability of the formulation, as well as be beneficial in the manufacturing process. Advantageous classes of excipients according to the invention include antiadherents, fillers, flavors, colorants, lubricants, preservatives, sweeteners carriers, solvents, solubilizing agents, buffers, preservatives, diluents, opacifiers.

It can be advantageous, respectively mandatory to add one or more pharmaceutically acceptable carrier to a pharmaceutically active agent. Eligible are all carriers known in the art and combinations thereof. In solid dosage forms they can be for example plant and animal fats, waxes, paraffins, starch, tragacanth, cellulose derivatives, polyethylene glycols, silicones, bentonites, silica, talcum, zinc oxide. For liquid dosage forms and emulsions suitable carriers are for example solvents, solubilizing agents, emulsifiers such as water, ethanol, isopropanol, ethyl carbonate, ethyl acetate, benzyl alcohol, benzyl benzoate, propylene glycol, 1,3-butyl glycol, cotton seed oil, peanut oil, olive oil, castor oil, sesame oil, glycerol fatty acid esters, polyethyl glycols, fatty acid esters of sorbitan. Suspensions according to the invention may use carriers known in the art such as diluents (e.g. water, ethanol or propylene glycol), ethoxylized isostearyl alcohols, polyoxyethylene and polyoxyethylene sorbitan esters, microcrystalline cellulose, bentonites, agar agar, tragacanth.

The term binding agents refers to substances that bind powders or glue them together, rendering them cohesive through granule formation. They serve as a "glue" of the formulation. Binding agents increase the cohesive strength of the provided diluent or filler.

Suitable binding agents are starch from wheat, corn, rice or potato, gelatine, naturally occurring sugars such as glucose, sucrose or beta-lactose, sweeteners from corn, natural and synthetic gums such as acacia, tragacanth or ammonium calcium alginate, sodium alginate, carboxymethyl cellulose, sodium carboxymethyl cellulose, hydroxypropyl carboxymethyl cellulose, polyethylene glycol, polyvinyl pyrrolidone, magnesium aluminium silicate, waxes and others. The percentage of the binding agent in the composition can range from 1 - 30 % by weight, preferred 2 - 20 % by weight, more preferred 3 - 10 % by weight and most preferred 3 - 6 % by weight.

In some embodiments it may be desirable that the prepared beverage generates some foam on being dissolved. Such an effect can be supported through the addition of a foaming agent that reduces the surface tension of the liquid, thus facilitating the formation of bubbles, or it increases its colloidal stability by inhibiting coalescence of bubbles. Alternatively, it may stabilize foam. Suitable examples include mineral oil, quillaia extract, triethyl citrate, sodium lauryl ether sulfate, sodium lauryl sulfate, ammonium lauryl sulfate.

Alternatively, some solubilisates according to the invention may appear slightly foamy upon preparation. Though this does not interfere with the desired application it may affect patient compliance in case of a medication or the commercial success in case of dietary supplements. Therefore it may be desirable to add a pharmaceutically or nutritionally acceptable anti-foaming agent (defoamer) to the solubilisate. Examples are polydimethylsiloxane or silicone oil in dietary supplements or simethicone in pharmaceuticals.

Thus the present application refers also to a beverage preparation capsule, in which a nutritionally and/or pharmaceutically acceptable antifoaming agent is added to the at least one dietary supplement and/or pharmaceutically active agent.

Lubricants are materials that prevent a baking of the respective supplements and improve the flow characteristics of granulations so that the flow is smooth and constant.

Suitable lubricants comprise silicon dioxide and talcum. The amount of the lubricant in the composition may vary between 0.01 and 10 % per weight, preferred between 0.1 and 7 % per weight, more preferred between 0.2 and 5 % per weight, most preferred between 0.5 and 2 % per weight.

Colorants are excipients that bestow a colorization to the composition of the drink, respectively the dosage form. These excipients can be food colorants. They can be adsorbed on a suitable adsorption means such as clay or aluminium oxide. The amount of the colorant may vary between 0.01 and 10 % per weight of the composition, preferred between 0.05 and 6 % per weight, more preferred between 0.1 and 4 % per weight, most preferred between 0.1 and 1 % per weight.

Suitable food colorants are curcumin, riboflavin, riboflavin-5'-phosphate, tartrazine, alkannin, quinolione yellow WS, Fast Yellow AB, riboflavin-5'-sodium phosphate, yellow 2G, Sunset yellow FCF, orange GGN, cochineal, carminic acid, citrus red 2, carmoisine, amaranth, Ponceau-4R, Ponceau SX, Ponceau 6R, erythrosine, red 2G, Allura red AC, Indathrene blue RS, Patent blue V, indigo carmine, Brilliant blue FCF, chlorophylls and chlorophyllins, copper complexes of chlorophylls and chlorophyllins, Green S, Fast Green FCF, Plain caramel, Caustic sulphite caramel, ammonia caramel, sulphite ammonia caramel, Black PN, Carbon black, vegetable carbon, Brown FK, Brown HT, alpha-carotene, beta-carotene, gamma-carotene, annatto, bixin, norbixin, paprika oleoresin, capsanthin, capsorubin, lycopene, beta-apo-8'-carotenal, ethyl ester of beta-apo-8'-carotenic acid, flavoxanthin, lutein, cryptoxanthin, rubixanthin, violaxanthin, rhodoxanthin, canthaxanthin, zeaxanthin, citranaxanthin, astaxanthin, betanin, anthocyanins, saffron, calcium carbonate, titanium dioxide, iron oxides, iron hydroxides, aluminium, silver, gold, pigment rubine, tannin, orcein, ferrous gluconate, ferrous lactate.

Flavor enhancers are widely used for food and drinks. Suitable examples are glutamic acid, monosodium glutamate, monopotassium glutamate, calcium diglutamate, monoammonium glutamate, magnesium diglutamate, guanylic acid, sodium guanylate, disodium guanylate, dipotassium guanylate, calcium guanylate, inosinic acid, disodium inosinate, dipotassium inosinate, calcium inosinate, calcium 5'-ribonucleotides, disodium 5'-ribonucleotides, glycine, sodium glycinate, zinc acetate, gum benzoic, thaumatin, glycyrrhizin, neohesperidine dihydrochalcone, glyceryl monoacetate, glyceryl diacetate.

Moreover, buffer solutions are preferred for liquid formulations, in particular for pharmaceutical liquid formulations. The terms buffer, buffer system and buffer solution, in particular of an aqueous solution, refer to the capacity of the system to resist a pH change by the addition of an acid or a base, or by dilution with a solvent. Preferred buffer systems may be selected from the group comprising formate, lactate, benzoic acid, oxalate, fumarate, aniline, acetate buffer, citrate buffer, glutamate buffer, phosphate buffer, succinate, pyridine, phthalate, histidine, MES (2-(N-morpholino) ethanesulfonic acid, maleic acid, cacodylate (dimethyl arsenate), carbonic acid, ADA (N-(2-acetamido)imino diacetic acid, PIPES (4-piperazine-bis-ethanesulfonic acid), BIS-TRIS propane (1,3-bis[tris(hydroxymethyl)mehylaminol] propane), ethylene diamine, ACES (2-[(amino-2-oxoethyl)amino]ethanesulfonic acid), imidazol, MOPS (3-(N-morphino)-propanesulfonic acid, diethyl malonic acid, TES (2-[tris(hydroxymethyl)methyl]aminoethanesulfonic acid, HEPES (N-2-hydroxyethylpiperazine-N'-2-ethanesulfonic acid), as well as other buffers with a pKₐ between 3.8 and 7.7.

Preferred are carbonic acid buffers such as acetate buffer and dicarboxylic acid buffers such as fumarate, tartrate and phthalate as well as tricarboxylic acid buffers such as citrate.

A further group of preferred buffers are inorganic buffers such as sulfate hydroxide, borate hydroxide, carbonate hydroxide, oxalate hydroxide, calcium hydroxide and phosphate buffers. Another group of preferred buffers are nitrogen-containing puffers such as imidazol, diethylene diamine and piperazine. Furthermore preferred are sulfonic acid buffers such as TES, HEPES, ACES, PIPES, [(2-hydroxy-1,1-bis-(hydroxymethyl)ethyl)amino]-1-propanesulfonic acid (TAPS), 4-(2-hydroxyethyl)piperazine-1-propanesulfonic acid (EEPS), 4-morpholino-propanesulfonic acid (MOPS) and N,N-bis-(2-hydroxyethyl)-2-aminoethanesulfonic acid (BES). Another group of preferred buffers are glycine, glycyl-glycine, glycyl-glycyl-glycine, N,N-bis-(2-hydroxyethyl)glycine and N-[2-hydroxy-1,1-bis(hydroxymethyl)ethyl]glycine (tricine). Preferred are also amino acid buffers such as glycine, alanine, valine, leucine, isoleucine, serine, threonine, phenylalanine, tyrosine, tryptophan, lysine, arginine, histidine, aspartate, glutamate, asparagine, glutamine, cysteine, methionine, proline, 4-hydroxy proline, N,N,N-trimethyllysine, 3-methyl histidine, 5-hydroxy-lysine, o-phosphoserine, gamma-carboxyglutamate, [epsilon]-N-acetyl lysine, [omega]-N-methyl arginine, citrulline, ornithine and their derivatives.

Preservatives for liquid dosage forms or supplements can be used on demand. They may be selected from the group comprising sorbic acid, potassium sorbate, sodium sorbate, calcium sorbate, methyl paraben, ethyl paraben, methyl ethyl paraben, propyl paraben, benzoic acid, sodium benzoate, potassium benzoate, calcium benzoate, heptyl p-hydroxybenzoate, sodium methyl para-hydroxybenzoate, sodium ethyl para-hydroxybenzoate, sodium propyl para-hydroxybenzoate, benzyl alcohol, benzalkonium chloride, phenylethyl alcohols, cresols, cetylpyridinium chloride, chlorobutanol, thiomersal (sodium 2-(ethylmercurithio) benzoic acid), sulfur dioxide, sodium sulphite, sodium bisulphite, sodium metabisulphite, potassium metabisulphite, potassium sulphite, calcium sulphite, calcium hydrogen sulphite, potassium hydrogen sulphite, biphenyl, orthophenyl phenol, sodium orthophenyl phenol, thiabendazole, nisin, natamycin, formic acid, sodium formate, calcium formate, hexamine, formaldehyde, dimethyl dicarbonate, potassium nitrite, sodium nitrite, sodium nitrate, potassium nitrate, acetic acid, potassium acetate, sodium acetate, sodium diacetate, calcium acetate, ammonium acetate, dehydroacetic acid, sodium dehydroacetate, lactic acid, propionic acid, sodium propionate, calcium propionate, potassium propionate, boric acid, sodium tetraborate, carbon dioxide, malic acid, fumaric acid, lysozyme, copper-(II)-sulfate, chlorine, chlorine dioxide and other suitable substances or compositions known to the person skilled in the art.

Additional emulsifiers can be selected for example from the following anionic and non-ionic emulsifiers: Anionic emulsifier waxes, cetyl alcohol, cetylstearyl alcohol, stearic acid, oleic acid, polyoxyethylene polyoxypropylene block polymers, addition products of 2 to 60 mol ethylene oxide to castor oil and/or hardened castor oil, wool wax oil (lanolin), sorbitan esters, polyoxyethylene alkyl esters, polyoxyethylene sorbitan fatty acid esters, polyoxyethene sorbitan monolaurate, polyoxyethene sorbitan monooleate, polyoxyethene sorbitan monopalmitate, polyoxyethene sorbitan monostearate, polyoxyethene sorbitan tristearate, polyoxyethene stearate, polyvinyl alcohol, metatartaric acid, calcium tartrate, alginic acid, sodium alginate, potassium alginate, ammonium alginate, calcium alginate, propane-1,2-diol alginate, carrageenan, processed eucheuma seaweed, locust bean gum, tragacanth, acacia gum, karaya gum, gellan gum, gum ghatti, glucomannane, pectin, amidated pectin, ammonium phosphatides, brominated vegetable oil, sucrose acetate isobutyrate, glycerol esters of wood rosins, disodium phosphate, trisodium diphosphate, tetrasodium diphosphate, dicalcium diphosphate, calcium dihydrogen diphosphate, sodium triphosphate, pentapotassium triphosphate, sodium polyphosphates, sodium calcium polyphosphate, calcium polyphosphates, ammonium polyphosphate, beta-cyclodextrin, powdered cellulose, methyl cellulose, ethyl cellulose, hydroxypropyl cellulose, hydroxypropyl methylcellulose, ethyl methyl cellulose, carboxymethyl cellulose, sodium carboxymethyl cellulose, ethyl hydroxyethyl cellulose, crosscarmellose, enzymically hydrolyzed carboxymethyl cellulose, mono- and diglycerides of fatty acids, glyceryl monostearate, glyceryl distearate, acetic acid esters of mono- and diglycerides of fatty acids, lactic acid esters of mono- and diglycerides of fatty acids, citric acid esters of mono- and diglycerides of fatty acids, tartaric acid esters of mono- and diglycerides of fatty acids, mono- and diacetyl tartaric acid esters of mono- and diglycerides of fatty acids, mixed acetic and tartaric acid esters of mono- and diglycerides of fatty acids, succinylated monoglycerides, sucrose esters of fatty acids, sucroglycerides, polyglycerol esters of fatty acids, polyglycerol polyricinoleate, propane-1,2-diol esters of fatty acids, propylene glycol esters of fatty acids, lactylated fatty acid esters of glycerol and propane-1, thermally oxidized soy bean oil interacted with mono- and diglycerides of fatty acids, dioctyl sodium sulphosuccinate, sodium stearoyl-2-lactylate, calcium stearoyl-2-lactylate, stearyl tartrate, stearyl citrate, sodium stearoyl fumarate, calcium stearoyl fumarate, stearyl tartrate, stearyl citrate, sodium stearoyl fumarate, calcium stearoyl fumarate, sodium laurylsulfate, ethoxylated mono- and diglycerides, methyl glucoside-coconut oil ester, sorbitan monostearate, sorbitan tristrearate, sorbitan monolaurate, sorbitan monooleate, sorbitan monopalmitate, sorbitan trioleate, calcium sodium polyphosphate, calcium polyphosphate, ammonium polyphosphate, cholic acid, choline salts, distarch glycerol, starch sodium octenyl succinate, acetylated oxidized starch.

Preferred are glycerin monooleate and stearic acid.

Stabilizers are substances that can be added to prevent unwanted changes. Though stabilizers are not real emulsifiers they may also contribute to the stability of emulsions, respectively solubilisates. Suitable examples for stabilizers are oxystearin, xanthan gum, agar, oat gum, guar gum, tara gum, polyoxyethene stearate, aspartame-acesulfame salt, amylase, proteases, papain, bromelain, ficin, invertase, polydextrose, polyvinyl pyrrolidone, polyvinyl polypyrrolidone, triethyl citrate, maltitol, maltitol syrup.

Suitable as additional surface-active solubilizing agents (solubilizers) are for example diethylene glycol monoethyl ester, polyethyl propylene glycol co-polymers, cyclodextrins such as α- and β-cyclodextrin, glyceryl monostearates such as Solutol HS 15 (Macrogol-15-hydroxystearate from BASF, PEG 660-15 hydroxystearates), sorbitan esters, polyoxyethylene glycol, polyoxyethylene sorbitanic acid esters, polyoxyethylene sorbitan monooleate, polyoxyethylene oxystearic acid triglyceride, polyvinyl alcohol, sodium dodecyl sulfate, (anionic) glyceryl monooleates etc.

Suitable aromatic and flavoring substances comprise above all essential oil that can be used for this purpose. In general, this term refers to volatile extracts from plants or parts of plants with the respective characteristic smell. They can be extracted from plants or parts of plants by steam distillation.

Examples are: Essential oils, respectively aromatic substances from sage, cloves, chamomile, anise, star anise, thyme, tea tree, peppermint, mint oil, menthol, cineol, eucalyptus oil, mango, figs, lavender oil, chamomile blossoms, pine needles, cypress, oranges, rosewood, plum, currant, cherry, birch leaves, cinnamon, limes, grapefruit, tangerine, juniper, valerian, lemon balm, lemon grass, palmarosa, cranberry, pomegranate, rosemary, ginger, pineapple, guava, echinacea, ivy leave extract, blueberry, kaki, melons etc. or mixtures thereof, as well as mixtures of menthol, peppermint and star anise oil or menthol and cherry flavor.

These aromatic or flavoring substances can be included in the range of 0.0001 to 10 % per weight (particularly in a composition), preferred 0.001 to 6% per weight, more preferred 0.001 to 4% per weight, most preferred 0.01 to 1% per weight, with regard to the total composition. Application- or single case-related it may be advantageous to use differing quantities.

Suitable sweeteners can be selected from the group comprising mannitol, glycerol, acesulfame potassium, aspartame, cyclamate, isomalt, isomaltitol, saccharin and its sodium, potassium and calcium salts, sucralose, alitame, thaumatin, glycyrrhizin, neohesperidine dihydrochalcone, steviol glycosides, neotame, aspartame-acesulfame salt, maltitol, maltitol syrup, lactitol, xylitol, erythritol.

Suitable solvents may be selected from the group comprising water, carbonated water, water for injection, water with isotonizing agents, saline, isotonic saline, alcohols, particularly ethyl and n-butyl alcohol, glycols, oleic and linoleic acid triglycerides, caprylic and capric acid mono-, di- and triglycerides, polyoxyethylene caprylic and capric acid glycerides, propylene glycol fatty acid esters, low alkyl fatty acid esters, soy bean oil, propylene glycol laurate, polyoxyethylene (35) castor oil, polyoxyethylene glyceryl trioleate, ethyl butyrate, ethyl caprylate, ethyl oleate and mixtures thereof.

Suitable isotonizing agents are for example pharmaceutically acceptable salts, in particular sodium chloride and potassium chloride, sugars such as glucose or lactose, sugar alcohols such as mannitol and sorbitol, citrate, phosphate, borate and mixtures thereof.

Suitable thickening agents can be selected from the group comprising polyvinyl pyrrolidone, methyl cellulose, hydroxypropyl methyl cellulose, hydroxypropyl cellulose, dextrins, polydextrose, modified starch, alkaline modified starch, bleached starch, oxidized starch, enzyme-treated starch, monostarch phosphate, distarch phosphate esterified with sodium trimetaphosphate or phosphorus oxychloride, phosphate distarch phosphate, acetylated distarch phosphate, starch acetate esterified with acetic anhydride, starch acetate esterified with vinyl acetate, acetylated distarch adipate, acetylated distarch glycerol, distarch glycerin, hydroxy propyl starch, hydroxy propyl distarch glycerine, hydroxy propyl distarch phosphate, hydroxy propyl distarch glycerol, starch sodium octenyl succinate, acetylated oxidized starch, hydroxyethyl cellulose.

Diluents or fillers are inactive substances added to drugs in order to handle minimal amounts of active agents. They can be useful in the solubilizing process. Examples for suitable diluents are water, mannitol, pre-gelatinized starch, starch, microcrystalline cellulose, powdered cellulose, silicified microcrystalline cellulose, dibasic calcium phosphate dihydrate, calcium phosphate, calcium carbonate, hydroxypropyl cellulose, hydroxyethyl cellulose, hydroxypropyl methylcellulose, polyethylene glycol, xanthum gum, gum arabic or any combination thereof.

Opacifiers are substances that render the drinkable liquid opaque, if desired. They must have a refractive index substantially different from the solvent, in most cases here water. At the same time they should be inert to the other components of the composition. Suitable examples include titanium dioxide, talc, calcium carbonate, behenic acid, cetyl alcohol, or mixtures thereof.

According to the invention all of the aforementioned excipients and classes of excipients can be used without limitation alone or in any conceivable combination thereof, as long as the inventive use of a solubilisate is not thwarted, toxic actions may occur or the respective national legislations are infracted.

Thus the present application refers also to a beverage preparation capsule, in which the solubilisate contains at least one pharmaceutically active agent, for use as a dosage form in medicine.

As can be learnt from the preparation instructions from Examples 1 - 10 standard size beverage preparation capsules aren't always needed for the purposes of the present invention. Often a capsule with significantly less volume would be sufficient to host the required volume for a solubilisate of a dietary supplement or a pharmaceutically active agent according to the invention. The dimensions of the mostly used beverage preparation capsules range between 17 mm (Tassimo standard size, Lavazzo Amodo Mio) and 35.3 mm (Hyperespresso System) in height, between 30 mm (Nespresso) and 63.3 mm (Tassimo large size) in diameter (fit), between 2 mm and 8.5 mm additionally for the flange and between 1 mm and 3.9 mm for the thickness of the flange.

Thus it is an additional task of the present application to provide small volume beverage preparation capsules. They take up less space, a great advantage in transporting and storing on a commercial scale, and they require less material. Therefore such capsules can be produced for less costs and offered at a better price. Also the consumer profits from such capsules, as they can be transported much easier. Such capsules will be referred to as minicapsules throughout this application.

Useful dimensions for these minicapsules are:
height: 5 mm to 12 mm, preferred 6 mm to 10 mm, most preferred 8 mm to 10 mm;
diameter: 5 mm to 25 mm, preferred 10 mm to 20 mm, most preferred 15 mm to 20 mm.

For reasons of practicability, process safety and material safety the dimensions of the flange and the thickness of the flange are the same as in the mentioned beverage preparations known in the art.

Thus the present application refers also to beverage preparation capsule, in which said capsule is a minicapsule, having a diameter from 5 mm to 25 mm and a height from 5 mm to 12 mm.

In particular, the present application refers also to beverage preparation capsule, in which said capsule is a minicapsule, having a diameter from 5 mm to 25 mm and a height from 5 mm to 12 mm, filled with a solubilisate of at least one dietary supplement and/or pharmaceutically active agent.

The present application also refers to the use of a minicapsule, having a diameter from 5 mm to 25 mm and a height from 5 mm to 12 mm, for the preparation of a beverage, in particular to the use of a minicapsule, having a diameter from 5 mm to 25 mm and a height from 5 mm to 12 mm, filled with a solubilisate of at least one dietary supplement and/or pharmaceutically active agent, for the preparation of a beverage.

It is understood that all specifications and modifications mentioned in this application for standard beverage preparation capsules shall also refer to these minicapsules.

In case any modifications in the beverage dispenser itself will be necessary for the use of these minicapsules, in particular in the beverage preparation capsule holder, tubing, nozzle diameters and process software, these modifications will be in the realm of knowledge of a person skilled in the art familiar with the construction of such beverage dispensers.

The present application also discloses a beverage dispensing system, comprising
a) a beverage dispenser for preparing and dispensing a beverage from a beverage preparation capsule,
b) a beverage preparation capsule according to the invention, and
c) a beverage container.

In another embodiment of the invention the beverage dispensing system according to the invention comprises means for avoiding or at least significantly reducing foaming when the prepared beverage is released from the beverage preparation capsule into the beverage container. As described before, unwanted foam may be generated thereby. This may affect patient compliance in case of a medication or the commercial success in case of dietary supplements. Defoaming elements such as the percolator-like inserts known from WO 2004/087529 A1 or WO 2005/018395 A1 (see above) showed were not completely satisfactory for the solubilisates from the beverage preparation capsules according to the invention, in particular when the solubilisate is slightly viscous, in comparison to mainly water-based beverages.

It was found that such foaming can be reliably avoided by letting the beverage pass through a flow restriction element on its way from the capsule into the container. Such a flow restriction element can be attached into or onto a release tube or funnel (in case of a beverage preparation machine having such a release tube or funnel) or into or onto the bottom of a beverage preparation capsule. Useful flow restriction elements comprise a thread in the lumen of a tube (a left-handed or a right-handed thread), an orifice plate or disk (a plate or disk with a central recess) inside a tube or at the lower end of a tube, perpendicular to the direction of flow of the beverage, or simply a nozzle inside a tube or at the lower end of a tube (distally tapered nozzle). All these restriction elements can be made from any suitable inert and heat-resisting material (until 100°C) such as suitable thermoplastics or steel. Ideally, the selected material should match the material of the tube or funnel, or of the beverage preparation capsule. Preferred are embodiments in polypropylene or medical stainless steel. It is inside the knowledge of a person skilled in the art to find out the required dimensions of such a flow restriction element and how to mount it reliably into or onto a release tube or funnel or the bottom of a beverage preparation capsule (cf. Examples 4 and 6).

Thus the present application also refers to a beverage dispensing system, wherein said beverage dispenser and/or said beverage preparation capsule comprises a tube or a nozzle for the outlet of said beverage into said beverage container, the tube or nozzle being characterized by containing a flow restriction device. In preferred embodiments this flow restriction element is a thread, an orifice plate or a distally tapered nozzle.

From the art beverage dispensing machines are known that are equipped with a rotating table (revolver disk) able to accommodate several beverage preparation capsules at a time. By simple revolving this table a new beverage preparation capsule is brought into the correct position for preparation of the beverage. Such a rotating table serves to ease the loading process, in particular if there is a high throughput of beverage preparations such as in cafés or in companies, or if the beverage to be prepared can be selected from beverage preparation capsules with a different content.

In a further embodiment of the invention such a rotating table can be configured to accommodate a number of minicapsules by simply modifying the recess diameter to the diameter of the selected minicapsules.

Thus the present application also refers to a beverage dispensing system, in which a beverage dispenser according to the invention is equipped with or configured to receive a beverage preparation capsule holder or beverage preparation capsule rotating table configured to receive at least one minicapsule according to the invention.

Thus the present application also refers to rotating table or revolver disk that is to be received in the beverage dispenser of a beverage dispensing system according to the invention, having a plurality of recesses for receiving beverage preparation capsules, wherein said recesses are configured to receive beverage preparation capsules of one or more sizes, at least one of these recess sizes being configured to receive a minicapsule according to the invention.

Sophisticated beverage dispensers known from the art are able to recognize from which beverage preparation capsule should be prepared next. This is particularly useful in case the beverage to be prepared can be selected from beverage preparation capsules of several different sizes and/or tastes. The user can select his preferred beverage via a computer-controlled display, as known in the art. The selected beverage preparation capsule type is recognized by the beverage dispenser through a machine-readable bar code printed on the upper side or the lower side of the respective beverage preparation capsule from where it can be read out by a suitable bar code reading unit of the beverage dispensing system placed in a suitable position. A computer program translates the bar code(s) to a display on the user surface, or lights a lamp at the correct side of a pre-selected list of possible varieties. The user can also command the rotating table to revolve to the next position until he has found his favorite taste or size. Alternatively, the same goal can be achieved by using RFID chip technology. Herein an RFID chip is attached into or onto the respective beverage preparation capsule, and by a RFID reading unit of the beverage dispenser the respective varieties of beverage preparation capsules at offer can be read out and displayed to the user as described before. Such beverage preparation capsule identification systems can also be used for the inventive beverage preparation capsules containing a solubilisate, in particular for minicapsules.

Thus the present application refers also to a beverage dispensing system, in which the beverage preparation capsules are provided with a machine-readable bar code on or a RFID chip in or on at least one of the external capsule sides, the beverage dispenser is provided with a matching bar code or RFID chip reader, and said bar code or said RFID chip being specific for the solubilisate contained in this beverage preparation capsule and for the size of said beverage preparation capsule.

In a preferred embodiment the beverage dispenser is configured to set its variable operational parameters by default according to the information read out from said bar code or RFID chip.

The present application also refers to a method for preparing a beverage by means of the beverage dispensing system according to the invention, comprising the following steps:
a) providing a beverage preparation capsule according to the invention;
b) placing said capsule in said beverage dispenser,
c) placing a conveniently sized beverage container below the nozzle of the beverage dispenser; and
d) operating the mechanism of the beverage dispensing system.

The present application also refers to a beverage, produced by the beverage dispensing system according to the invention.

In a particularly preferred embodiment of said beverage the solubilisate contains a pharmaceutically active agent for use in medicine.

### Examples

### Example 1:

In order to generate a solubilisate of the loop diuretic furosemide (a BCS class IV pharmaceutical drug) 4.5 g *Poloxamer* 188 and 1.25 g α-tocopherol were heated up to 60°C until they melt. 4.76 ml aqua bidest (25%) were heated to 60°C and used to cover the molten mixture. It was waited until a gel was formed. In a second solution 14.29 ml aqua bidest (75%) were provided and 0.2 g furosemide were added under stirring. Then this second solution was added to the gel (for the method cf. WO 2007/104173 A2). Thus a solubilisate is yielded which is apt to form stable micelles enclosing the compound to be solubilized. As the indicated amount of aqua bidest is needed therefor it has to be regarded as a solubilisate and not as a concentrate. This solubilisate (25 ml) had approximatively the following composition:

| | |
|---|---|
| *Poloxamer 188* | 18 wt% |
| α-tocopherol | 5 wt% |
| furosemide | 0.8 wt% |
| aqua bidest | 76.2 wt% |

This solubilisate was used for refilling a commercially available beverage preparation capsule. A used Tassimo T Disk coffee capsule (standard size; 63.3 mm diameter, 17.0 mm height) was washed thoroughly with aqua bidest. The capsule was refilled by using a standard technique for refilling coffee capsules known in the art. 5 ml of the solubilisate were drawn up in a standard 10 ml syringe, as used for injections, and injected through the opening of the capsule. The charged capsule was inserted into a Tassimo machine from Bosch, a cup was placed under the outlet tube and the brewing was started. This yielded a beverage containing 40 mg furosemide, a standard dosage of this drug. The experiment was repeated two times (n = 3). Each time a clear solution without any slurs was generated. No compound sedimented, flocculated or re-crystallized upon cooling down of the beverage to room temperature during a two hours observation period.

### Example 2:

In order to generate a solubilisate of the very lipophilic dietary supplement β-carotene (a precursor of vitamin A, a terpenoid contained in a variety of plants such as carrots, pumpkins etc.) 4.5 g *Poloxamer* 188 and 1.25 g α-tocopherol were heated up to 60°C until they melt, as in Example 1. 4.81 ml aqua bidest (25%) were heated to 60°C and used to cover the molten mixture. It was waited until a gel was formed. In a second solution 14.41 ml aqua bidest (75 %) were provided and 30 mg β-carotene were added under stirring. Then this second solution was added to the gel. Thus a solubilisate is yielded which is apt to form stable micelles enclosing the compound to be solubilized when added to any quantity of water. This solubilisate (25 ml) had approximatively the following composition:

| | |
|---|---|
| *Poloxamer 188* | 18 wt% |
| α-tocopherol | 5 wt% |
| β-carotene | 0.12 wt% |
| aqua bidest | 76.88 wt% |

This solubilisate was used for refilling a commercially available beverage preparation capsule. The same type of Tassimo T Disk coffee capsules was refilled with 5 ml each of the solubilisate and a beverage prepared, as described in Experiment 1 (n = 3). This yielded a beverage containing 6 mg β-carotene, a recommended daily dosage for this dietary supplement. Each time a clear mildly orange-colored solution without any slurs was generated. No compound sedimented, flocculated or re-crystallized upon cooling down of the beverage to room temperature during a two hours observation period.

### Example 3:

In order to generate a solubilisate of the analgesic acetaminophen (paracetamol; a BCS class IV pharmaceutical drug) a 25 ml self-emulsifying composition was generated according to US 2006/0051642 A1. 5 g acetaminophen are provided in a sealable container. Then 15.7 ml ethyl oleate are added and the container sealed. The container is put in a water bath having a temperature of 50°C and shaken gently until all solid substance is dissolved. After cooling down the container to room temperature 1 ml octanoic acid, 3.23 ml Polysorbate 80, 50 mg of a mixture of butylated hydroxytoluene (BHT) and butylated hydroxyanisole (BHA), ratio 1:1 as well as 25 mg propyl gallate are sequentially added and stirred. Then the container is sealed again and gently shaken until a clear solution is formed. This solubilisate had approximatively the following composition:

| | |
|---|---|
| acetaminophen | 20 wt% |
| ethyl oleate | 62.8 wt% |
| octanoic acid | 4 wt% |
| Polysorbate 80 | 12.9 wt% |
| BHT/BHA | 0.2 wt% |
| propyl gallate | 0.1 wt% |

This solubilisate was used for refilling a commercially available beverage preparation capsule. A used Nescafé Dolce Gusto coffee capsule (standard size; 48.0 mm diameter, 34.5 mm height) was washed thoroughly with aqua bidest. The capsule was refilled by using a standard technique for refilling coffee capsules known in the art. 5 ml of the solubilisate were drawn up in a standard 10 ml syringe, as used for injections, and injected through the opening of the capsule. The charged capsule was inserted into a Nescafé Dolce Gusto compatible machine from Krups (Melody 3 Manual Coffee Machine), the lever was moved to cold water, a cup was placed under the outlet tube and the brewing was started. This yielded a beverage containing 1 g acetaminophen, a strong standard dosage of this drug. The experiment was repeated two times (n = 3). Each time a solution without any visible particles was generated. However, mild slurs occurred (zebra effect). No compound sedimented, flocculated or re-crystallized during a two hours observation period.

### Example 4:

Experiment 3 was repeated three times with the modification that a flow restriction element was placed onto the outlet of said coffee capsule. Therefore a polypropylene tube (1 cm length, 4 mm inner diameter) containing a left-handed thread was welded on the outlet of the capsule by means of a soldering iron. As a result, no slurs as in Example 3 occurred anymore.

### Example 5:

In order to generate a solubilisate of the hardly water-soluble dietary supplement melatonin a 25 ml self-emulsifying composition was generated, correspondingly to Example 3. 25 mg melatonin are provided in a sealable container. Then 19.63 ml ethyl oleate are added and the container sealed. The container is put in a water bath having a temperature of 50°C and shaken gently until all solid substance is dissolved. After cooling down the container to room temperature 1.25 ml octanoic acid, 4.03 ml Polysorbate 80, 50 mg of a BHT/BHA mixture ratio 1:1 as well as 25 mg propyl gallate are sequentially added and stirred. Then the container is sealed again and gently shaken until a clear solution is formed. This solubilisate had approximatively the following composition:

| | |
|---|---|
| melatonin | 0.1 wt% |
| ethyl oleate | 78.5 wt% |
| octanoic acid | 5 wt% |
| Polysorbate 80 | 16.1 wt% |
| BHT/BHA | 0.2 wt% |
| propyl gallate | 0.1 wt% |

This solubilisate was used for refilling a commercially available beverage preparation capsule. The same type of Nescafé Dolce Gusto coffee capsules was refilled with 5 ml each of the solubilisate and a cold beverage prepared, as described in Experiment 3 (n = 3). This yielded a beverage containing 5 mg melatonin, the maximum of the recommended daily dosage range for this dietary supplement. Each time a solution without any visible particles was generated. However, also with melatonin mild slurs occurred (zebra effect). No compound sedimented, flocculated or re-crystallized during a two hours observation period.

### Example 6:

Experiment 5 was repeated three times with the modification that a flow restriction element was placed onto the outlet of said coffee capsule. Therefore a polypropylene tube (1 cm length, 4 mm inner diameter) was welded on the outlet of the capsule by means of a soldering iron. Additionally, a perforated polypropylene disk (diameter of the central recess: 2 mm) was welded on the lower end of the tube in order to restrict the flow. As a result, no slurs as in Example 5 occurred anymore.

### Example 7:

25 ml of a liposome-based solubilisate of the sulfonylurea anti-diabetic drug glipizide (a BCS class IV pharmaceutical drug) were generated. The liposomes were produced from the phospholipid 1,2-dimyristoyl-sn-glycero-3-phosphocholine (DPMC). The phospholipid was solubilized by dissolving 50 mg DPMC in 2.5 ml t-butanol and heating the mixture in a 37°C water bath for 5 minutes. Then the solution was stored at -20°C in a tight container protected from light exposure. 50 mg glipizide were dissolved in 500 µl DMSO and also stored at -20°C in a tight container protected from light exposure. The next day the solutions were thawed and 2.5 ml of the DPMC solution, 500 µl of the glipizide solution and 22 ml t-butanol were thoroughly mixed (for liposome technology cf. US 2008/0103213 A1). This solubilisate had approximatively the following composition:

| | |
|---|---|
| glipizide | 0.2 wt% |
| DPMC | 0.2 wt% |
| DMSO | 2.0 wt% |
| t-butanol | 97.6 wt% |

This solubilisate was used for refilling a commercially available beverage preparation capsule. A used Nespresso coffee capsule (standard size; 30.0 mm diameter, 37.0 mm height) was washed thoroughly with aqua bidest. The capsule was refilled by using a standard technique for refilling coffee capsules known in the art. 5 ml of the solubilisate were drawn up in a standard 10 ml syringe, as used for injections, and injected through the opening of the capsule. The charged capsule was inserted into a Nespresso Inissia machine, a cup was properly placed and the brewing was started. This yielded a beverage containing 10 mg glipizide, a standard dosage of this drug. The experiment was repeated two times (n = 3). The solution was clear, slightly yellow and had a neutral acceptable taste.

### Example 8:

25 ml of a liposome-based solubilisate of folic acid (vitamin 8₉) were generated in analogy to Example 7. The liposomes were produced from the phospholipid 1,2-dimyristoyl-sn-glycero-3-phosphocholine (DPMC). The phospholipid was solubilized by dissolving 50 mg DPMC in 2.5 ml t-butanol and heating the mixture in a 37°C water bath for 5 minutes. Then the solution was stored at -20°C in a tight container protected from light exposure. 2 mg folic acid were dissolved in 500 µl DMSO and also stored at -20°C in a tight container protected from light exposure. The next day the solutions were thawed and 2.5 ml of the DPMC solution, 500 µl of the folic acid solution and 22 ml t-butanol were thoroughly mixed. This solubilisate had approximatively the following composition:

| | |
|---|---|
| folic acid | 0.008 wt% |
| DPMC | 0.2 wt% |
| DMSO | 2.0 wt% |
| t-butanol | 97.8 wt% |

5 ml of this solution were filled in a used Nespresso coffee capsule, as described before, and a beverage was prepared by means of Nespresso Inissia machine. This yielded a beverage containing 400 µg folic acid, a recommended daily dose for this dietary supplement. The experiment was repeated two times (n = 3). The solution was clear, slightly yellow and had a neutral acceptable taste.

### Example 9:

In order to generate a solubilisate of the antibiotic clarithromycin (a BCS class IV pharmaceutical drug) a 10 ml composition was generated. First, clarithromycin (2.5 g), β-cyclodextrin (70 mg) and fumaric acid (500 mg) were micronized and then dispersed together in a dispersion of the HPC in ware (7% w/w). A small amount of simethicone emulsion was added to defoam the dispersion. A blend of the non-ionic polymer hydroxypropylcellulose (630 mg) and unmicronized β-cyclodextrin (100 mg) was prepared in a planetary mixer. The clarithromycin dispersion was added to it and then the entire mixture is passed through an extruder. The resulting material was dried at 45°C. The dried exudate was milled again. Then it was dispersed in 6.2 ml aqua bidest. (for the method cf. US 2003/0091627 A1).

This solubilisate had approximatively the following composition:

| | |
|---|---|
| clarithromycin | 25 wt% |
| β-cyclodextrin | 1.7 wt% |
| fumaric acid | 5 wt% |
| hydroxypropylcellulose | 6.3 wt% |
| aqua bidest | 62 wt% |

This solubilisate was used for refilling a commercially available beverage preparation capsule. Lavazza Blue coffee capsules (standard size; 41 mm diameter, 26.6 mm height) were refilled with 2 ml each of the solubilisate and a beverage prepared, as described before (n = 3). As a beverage dispensing machine a Lavazza LB 951 was used. This yielded a beverage containing 500 mg clarithromycin, a standard dosage for antibiotic therapy. Each time a solution without any visible particles was generated. As many macrolide antibiotics, clarithromycin has a bitter taste. In the present formulation this taste is masked and the beverage has a slightly sweetish taste.

### Example 10:

In order to generate a solubilisate of the bioflavonoid quercetin for intake as a dietary supplement a 20 ml composition was generated. Similar to Example 9, quercetin (5 g), β-cyclodextrin (140 mg) and fumaric acid (1 g) were micronized first and then dispersed together in a dispersion of the HPC in ware (7% w/w). A small amount of simethicone emulsion was added to defoam the dispersion. A blend of the non-ionic polymer hydroxypropylcellulose (1.26 g) and unmicronized β-cyclodextrin (200 mg) was prepared in a planetary mixer. The quercetin dispersion was added to it and then the entire mixture is passed through an extruder. The resulting material was dried at 45°C. The dried exudate was milled again. Then it was dispersed in 12.4 ml aqua bidest.

This solubilisate had approximatively the following composition:

| | |
|---|---|
| quercetin | 25 wt% |
| β-cyclodextrin | 1.7 wt% |
| fumaric acid | 5 wt% |
| hydroxypropylcellulose | 6.3 wt% |
| aqua bidest | 62 wt% |

This solubilisate was used for refilling a commercially available beverage preparation capsule. Lavazza Blue coffee capsules were refilled with 4 ml each of the solubilisate and a beverage prepared, as described before (n = 3). Also herein the Lavazza LB 951 was used. This yielded a beverage containing 1 g quercetin, a recommended daily dosage. Each time a solution without any visible particles was generated. The bitter taste, typical for polyphenolic flavonoids such as quercetin was covered. The beverage has a slightly sweetish taste.

## Claims

1. A beverage preparation capsule containing a solubilisate of at least one pharmaceutically active agent and/or a dietary supplement.

2. The beverage preparation capsule according to claim 1, wherein the solubilisate is prepared from at least one poorly water-soluble substance or extract.

3. The beverage preparation capsule according to any claim 1 or 2, in which the solubilisate is prepared by means of micelle, liposome, self-emulsification or cyclodextrin complexation technology.

4. The beverage preparation capsule according to any of the preceding claims, in which the solubilisate of the at least one dietary supplement and/or pharmaceutically active agent enhances the resorption and/or bioavailability of at least one of said dietary supplements or pharmaceutically active agents.

5. The beverage preparation capsule according to claim 3 or 4, in which the solubilisate was prepared from a substance selected from a group comprising β-carotene, melatonin, folic acid and quercetin, if the substance is a dietary supplement, or selected from a group comprising furosemide, acetaminophen, glipizide and clarithromycin, if the substance is a pharmaceutically active agent.

6. The beverage preparation capsule according to any of claims 3 to 4, in which the solubilisate contains at least one pharmaceutically active agent, for use as a dosage form in medicine.

7. The beverage preparation capsule according to claim 3, in which a bitter or unpleasant taste of the at least one pharmaceutical drug or dietary supplement is masked by the solubilisate prepared by micelle, liposome, self-emulsification or cyclodextrin complexation technology.

8. The beverage preparation capsule according to any of the preceding claims, in which said capsule is a minicapsule, having a diameter from 5 mm to 25 mm and a height from 5 to 12 mm.

9. A beverage dispensing system, comprising
a) a beverage dispenser for preparing and dispensing a beverage from a beverage preparation capsule,
b) a beverage preparation capsule according to anyone of claims 1 to 10, and
c) a beverage container.

10. The beverage dispensing system according to claim 9, in which said beverage dispenser and/or said beverage preparation capsule comprises a tube or a nozzle for the outlet of said beverage into said beverage container, the tube or nozzle being **characterized by** containing a flow restriction device.

11. The beverage dispensing system according to claim 10, in which said flow restriction device is a thread, an orifice plate or a distally tapered nozzle.

12. The beverage dispensing system according to any of claims 9 to 11, in which said beverage dispenser is equipped with or configured to receive a beverage preparation capsule holder or a beverage preparation capsule rotating table configured to receive at least one minicapsule according to claim 10.

13. A method for preparing a beverage by means of the beverage dispensing system according to any of claims 9 to 12, comprising the following steps:
a) providing a beverage preparation capsule according to any of claims 1 to 8;
b) placing said capsule in said beverage dispenser,
c) placing a conveniently sized beverage container below the nozzle of the beverage dispenser; and
d) operating the mechanism of the beverage dispensing system.

14. A beverage, produced by the beverage dispensing system according to any of claims 9 to 12.

15. A beverage according to claim 14, wherein the solubilisate contains a pharmaceutically active agent, for use in medicine.
